# EUROPEAN PATENT APPLICATION

(11) **EP 4 755 452 A1**
(43) Date of publication of application: **10.06.2026**
(21) Application number: 24217783.0
(22) Date of filing: 05.12.2024
(51) Int. Cl.: A61P 25/18, A61P 25/24, C07D 295/135

(54) **IMPROVED PROCESS FOR THE PREPARATION OF CARIPRAZINE**

(71) Applicant: F.I.S.- Fabbrica Italiana Sintetici S.p.A., 36075 Montecchio Maggiore (VI) (IT)
(72) Inventor: De Poli, Matteo, 36075 Montecchio Maggiore (VI) (IT); Tyler, Andrew, 36075 Montecchio Maggiore (VI) (IT); Marcazzan, Gabriele, 36075 Montecchio Maggiore (VI) (IT)

(57) **Abstract**

Object of the present invention is an improved process for the preparation of Cariprazine compound of formula (1): 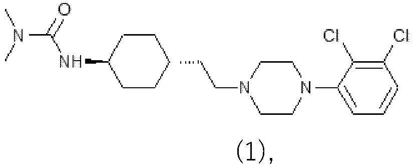 comprising the step of adding a base to a mixture comprising phosgene or a carbonic acid derivative and amino piperazine compound of formula (3): 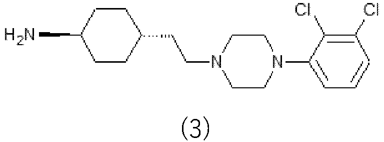 in a salt form, to give an isocyanate compound of formula (2): 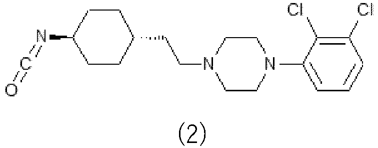 and/or compound of formula (11):

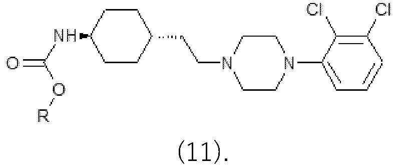

## Description

### Technical Field

The present invention refers to an improved process for the preparation of Cariprazine.

### Background Art

Cariprazine is a second-generation antipsychotic used in the treatment of schizophrenia, bipolar mania, bipolar depression, and major depressive disorders, which was approved for medical use in the United States in September 2015.

Cariprazine is an N-alkylpiperazine that is N,N-dimethyl-N'-{trans-4-[2-(piperazin-1-yl)ethyl]cyclohexyl}urea substituted at position 4 on the piperazine ring by a 2,3-dichlorophenyl group.

Its chemical name is 3-[trans-4-[2-[4-(2,3-dichlorophenyl)piperazin-1-yl] ethyl]cyclohexyl]-1,1-dimethylurea and has the following chemical formula (1):

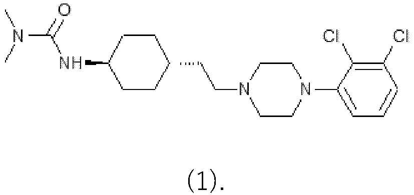

Cariprazine can be prepared according to several synthetic processes described in the literature.

The first synthesis of Cariprazine is described for example in WO 2005012266. The route of synthesis is reported in the following page. The synthesis starts with the reaction between 1-bromo-2,3-dichlorobenzene (9) and tertbutylpiperazine-1-carboxylate (8) using 2,2-bis-(diphenylphosphino)-1,1-binaphthyl (BINAP), tris-(dibenzylidene acetone)dipalladium(0) (Pd₂(dba)₃), and sodium tert-butoxide to afford tert-butyl-4-(2,3-dichlorophenyl)piperazine-1-carboxylate (7). Compound (7) is then submitted to Boc deprotection with gaseous hydrogen chloride to give 1-(2,3-dichlorophenyl)piperazine (6). Next, reductive amination of tert-butyl-((1R,4R)-4-(2-oxoethyl)cyclohexyl) carbamate (5) with compound (6) using sodium triacetoxyborohydride in dichloromethane under alkaline conditions at room temperature for 20 h gives tert-butyl ((1R,4R)-4-(2-(4-(2,3-dichlorophenyl)-piperazin-1-yl)ethyl)cyclohexyl)carbamate (4). Compound 4, in turn, is reacted with gaseous hydrogen chloride to give (1R,4R)-4-(2-(4-(2,3-dichlorophenyl) piperazin-1-yl)ethyl)cyclohexanamine hydrochloride (3). Triethylamine is then added over compound (3), followed by triphosgene. The isocyanate compound (2) thus obtained is finally reacted with dimethylamine to obtain Cariprazine (1).

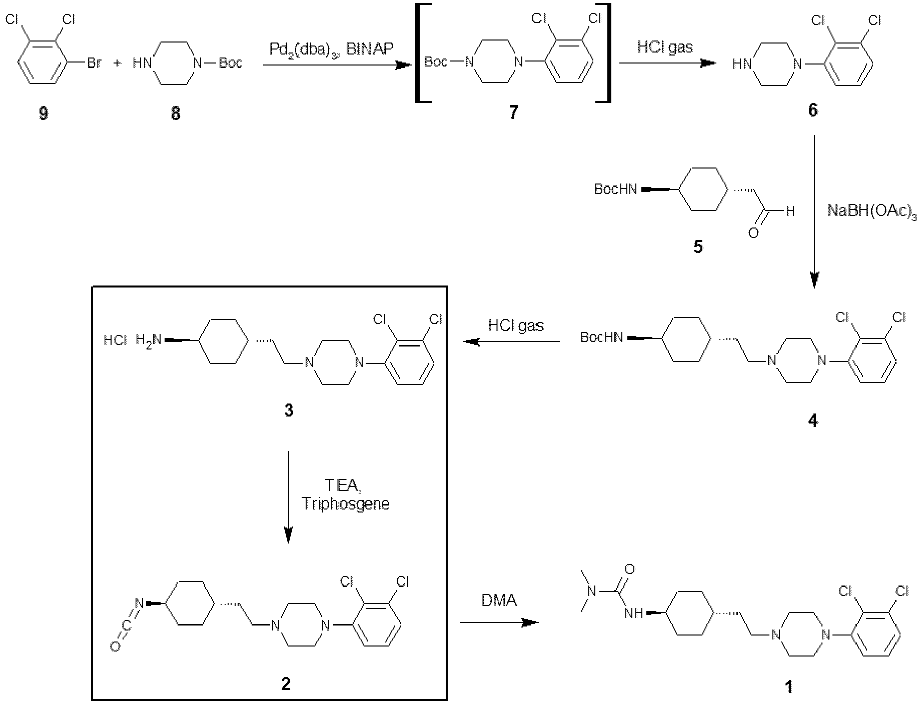

Main drawback of this route of synthesis is the poor yield obtained in the transformation of compound of formula (3) into compound of formula (2), which is reported in the patent application to be 52% molar (refer to WO2005012266, Method C at page 20). This low yield is mainly due to the uncomplete conversion of compound (3) and to the generation of many by-products, such as a dimer compound, during the reaction time.

This problem was overcome in WO2010070370 by performing the same reaction by adding a suspension of compound (3) and triethylamine in dichloromethane to a triphosgene solution in dichloromethane. Thanks to this improvement, the step yield was increased to 95% molar (refer to Example 3 at page 8 of the patent application).

This method, however, is not applicable at industrial scale, as the suspension of compound of formula (3) and triethylamine is so thick that the mixture cannot conveniently be transferred using common industrial equipment. Dosing such a thick suspension over the triphosgene solution as taught in WO2010070370 procedure, therefore, is only feasible at small or lab scale.

Moreover, said process is not suitable for the industrial manufacturing also because of the employment of dichloromethane (DCM) as solvent. It is well known, in fact, that DCM is listed among the solvents to be limited as far as possible in pharmaceutical products due to its toxicity. DCM should be avoided also for environmental reasons, as chlorinated solvents are frequently found to contaminate the groundwater system where they pose a serious threat to human health and the environment.

As a further drawback, the synthesis described in WC2010070370 requires a significant excess of the hazardous reactant triphosgene to allow the reaction to reach completion. Handling of great amounts of triphosgene represents another obstacle towards the industrial applicability of the above-mentioned procedure.

In addition, according to the procedure reported in WO2010070370, dimethylamine is employed in the last step of the process as a solution in isopropanol. From a solvent recovery perspective, increasingly important in the current industrial manufacturing, the recycling of organic solvents from mixtures of the same is much more complicated.

It clearly appears, therefore, that, despite the higher yield obtained, the process described in WO2010070370 is not suitable for industrial scale production of Cariprazine.

Thus, there is a need for a process for preparing Cariprazine which is suitable for industrial manufacturing, and thus adequate for standard industrial equipment, characterized by high yields and productivity and, at the same time, environmentally sustainable.

### Summary of the invention

The problem addressed by the present invention is therefore that of providing an improved process for the preparation of Cariprazine which simultaneously solve the following problems:
- being capable to produce Cariprazine with higher yield;
- being suitable for industrial manufacturing, and thus adequate for being carried out on standard industrial equipment.

As additional problem, it would be desirable to provide a process for the preparation of Cariprazine which is environmentally sustainable.

These problems are solved by a process for the preparation of Cariprazine compound of formula (1): 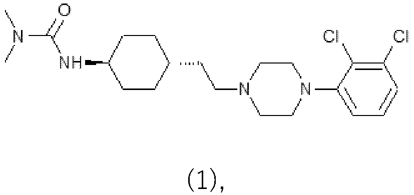 comprising the following steps:
a) adding a base to a mixture comprising:
   - amino piperazine compound of formula (3): 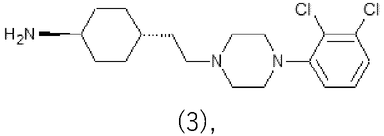 wherein said compound of formula (3) is in a salt form, and
   - phosgene or a carbonic acid derivative of formula (10):

      R-O-CO-Z (10),

      wherein R is a C₁₋₆ straight or branched alkyl chain or a C₁₋₂ fully halogenated alky and Z is O-R or X, wherein R is as described above and X is a halogen,
   to give an isocyanate compound of formula (2): 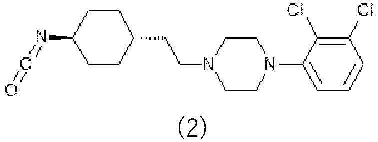 and/or compound of formula (11): 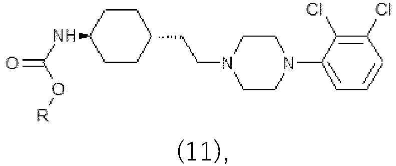 wherein R is as described above;
b) reacting compound of formula (2) and/or compound of formula (11) obtained in step a) with dimethylamine to give Cariprazine compound of formula (1).

Further features and advantages of the present invention will result from the description hereafter reported and from the annexed claims, whose definitions are integral part of the present description.

### Detailed description of the invention

The present invention is related to a process for preparing Cariprazine compound of formula (1): 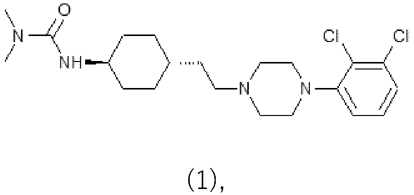 comprising the following steps:
a) adding a base to a mixture comprising:
   - amino piperazine compound of formula (3): 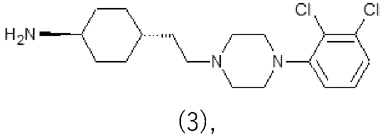 wherein said compound of formula (3) is in a salt form, and
   - phosgene or a carbonic acid derivative of formula (10):

      R-O-CO-Z (10),

      wherein R is a C₁₋₆ straight or branched alkyl chain or a C₁₋₂ fully halogenated alky and Z is O-R or X, wherein R is as described above and X is a halogen,
   to give an isocyanate compound of formula (2): 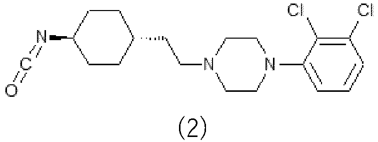 and/or compound of formula (11): 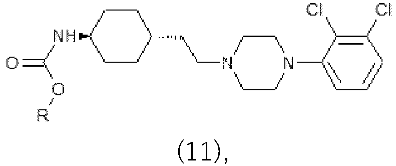 wherein R is as described above;
b) reacting compound of formula (2) and/or compound of formula (11) obtained in step a) with dimethylamine to give Cariprazine compound of formula (1).

It was indeed surprisingly found that, by adding a base over a mixture comprising compound of formula (3) as a salt and a carbonic acid derivative, as depicted for example in the scheme below, the problems found in prior art methods are overcome.

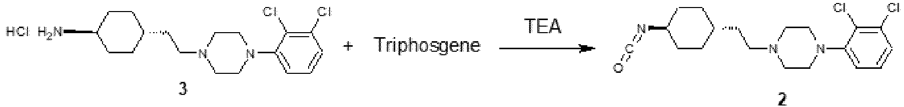

According to WO2005012266 method C, compound of formula (2) is obtained by dosing the carbonic acid derivative, triphosgene, over a mixture comprising a base, triethylamine, and compound of formula (3) as trihydrochloride salt. After reaction with dimethylamine, Cariprazine compound of formula (1) is obtained. This method was reproduced in Example 7 of the present application, resulting in a very poor yield (79% molar yield, 58.3%wt HPLC assay of the product, 46% molar yield corrected for the assay).

Following the procedure disclosed in WO2010070370 Example 3, compound of formula (2) is obtained by adding a suspension of compound of formula (3) as dihydrochloride salt and triethylamine in dichloromethane into a triphosgene solution in dichloromethane. After reaction with dimethylamine, Cariprazine compound of formula (1) is obtained. This method was reproduced in Example 8 of the present application, resulting in a better yield (98% molar yield, 93.3%wt HPLC assay of the product, 91% molar yield corrected for the assay). This procedure, however, was very difficult to be carried out, even at lab scale, due to the issues found in transferring the thick suspension comprising compound (3) and triethylamine.

By following the present application process, i.e. by adding triethylamine into a suspension of compound of formula (3) as dihydrochloride salt and triphosgene to obtain compound of formula (2), conversely, after reaction with dimethylamine, compound of formula (1) is obtained with an even better yield (97% average molar yield, 97%wt average HPLC assay of the product, 94% average molar yield corrected for the assay). Moreover, the issues found with WO2010070370 procedure when transferring the thick suspension was overcome as, following the process of the present application, the transfer involved is that of a solution or a liquid material into a suspension. Refer to comparative table of Example 9 of the present application for the clear effect provided by the present invention.

In all the procedures cited above, i.e. procedure as from WO2005012266 method C, procedure as from WO2010070370 Example 3, and procedure according to the present application, the role of the base is, first, that of capturing the anion or the anions of compound of formula (3) as a salt, and thus allowing to obtain compound of formula (3) as free amine. The second role of the base is also to neutralize any acid by-product resulting from the reaction of compound of formula (3) amine with the carbonic acid derivative of formula (10). It follows that the amount of the base to be charged according to all the procedures should be such as to allow said base to perform both the roles, i.e. freeing the primary amine function of compound of formula (3) and capturing any acid by-product.

The considerations reported above for the amount of base to be charged also applies to the present invention.

According to a preferred aspect of the process of the present invention, in step a) the base is triethylamine or diisopropylethylamine.

According to a more preferred aspect of the process of the present invention, in step a) the base is triethylamine.

According to another preferred aspect of the process of the present invention, in step a) the base is added over a period comprised between 30 minutes and 5 hours.

According to a more preferred aspect of the process of the present invention, in step a) the base is added over a period comprised between 30 minutes and 1 hour.

According to a preferred aspect of the process of the present invention, in step a) the base is added at a temperature comprised in the range from -30° C to 20° C.

According to a more preferred aspect of the process of the present invention, in step a) the base is added at a temperature comprised in the range from -20° C to 0° C.

According to a preferred aspect of the process of the present invention, in step a) the base is added over a period comprised between 30 minutes and 5 hours at a temperature comprised in the range from -30° C to 20° C.

According to a more preferred aspect of the process of the present invention, in step a) the base is added over a period comprised between 30 minutes and 1 hour at a temperature comprised in the range from - 20° C to 0° C.

According to a preferred aspect of the process of the present invention, in step a) the carbonic acid derivative is methylchloroformate.

According to a more preferred aspect of the process of the present invention, in step a) the carbonic acid derivative (10) is diphosgene or triphosgene.

According to an even more preferred aspect of the process of the present invention, in step a) the carbonic acid derivative (10) is triphosgene.

The term "weight" (W), as intended in the present application, means weight of reactant per unit of product, thus, for example, 1 W is 1 Kilo per 1 Kilo, or 1 gram per 1 gram, or 1 milligram per 1 milligram. Thus, 10 W means for example 10 grams per 1 gram of substance.

According to WO2005012266 method C procedure, only 0.26 W (with respect to compound of formula (3) as dihydrochloride salt) of triphosgene are employed to convert compound of formula (3) into compound of formula (2). This procedure, however, resulted in very poor yield, as stated above. According to WO2010070370 Example 3 procedure, on the other hand, 0.76 W of triphosgene (with respect to compound of formula (3) as dihydrochloride salt) are employed for the same conversion, achieving a higher process yield.

It was surprisingly found that, following the process of the present application, by employing 0.36 W - 0.55 W (with respect to compound of formula (3) as dihydrochloride salt) of triphosgene for the conversion of compound of formula (3) into compound of formula (2), the yield obtained is even better than that characterizing WO2010070370 Example 3 process. If considering molar yield corrected for product assay (HPLC wt%), in fact, 92-97% molar yield is obtained applying the present application process, while 91% yield results when reproducing WO2010070370 Example 3 process (refer to Example 10 of the present application).

It is well known that triphosgene is a hazardous reactant, which can be fatal if inhaled. Reducing the amount of reagent needed to reach reaction completion while maintaining at the same time high process performances is therefore an important goal when applying the process at industrial scale. The process of the present application allows to obtain even higher process yield (+3% average higher yield), while at the same time reducing by 30%-50% the amount of triphosgene needed to complete the reaction.

According to a preferred aspect of the process of the present invention, the reaction in step a) is carried out using 0.36 W - 0.55 W (with respect to compound of formula (3) as dihydrochloride salt) of triphosgene as carbonic acid derivative.

According to a more preferred aspect of the process of the present invention, the reaction in step a) is carried out using 0.36 W (with respect to compound of formula (3) as dihydrochloride salt) of triphosgene as carbonic acid derivative.

Both prior art processes disclosed in WO2005012266 method C and WO2010070370 Example 3 employ DCM as solvent for the conversion of compound of formula (3) into compound of formula (2).

Industrial manufacturing is well aware that DCM is listed among the solvents to be limited as far as possible in pharmaceutical products due to its toxicity. DCM should be avoided also for environmental reasons, as chlorinated solvents are frequently found to contaminate the groundwater system where they pose a serious threat to human health and the environment.

The process of the present invention is particularly suitable for industrial manufacturing also considering safety and sustainability aspects, thanks to the use of non-chlorinated solvents, specifically by avoiding DCM as solvent.

According to a preferred aspect of the process of the present invention, in fact, the reaction in step a) is carried out in a solvent selected from the list comprising 2-methyl-tetrahydrofuran, tetrahydrofuran, acetone, acetonitrile.

According to a preferred aspect of the process of the present invention, in fact, the reaction in step a) is carried out in an aprotic solvent.

According to a more preferred aspect of the process of the present invention, the reaction in step a) is carried out in 2-methyl-tetrahydrofuran, tetrahydrofuran or acetone.

According to an even more preferred aspect of the process of the present invention, the reaction in step a) is carried out in 2-methyl-tetrahydrofuran.

According to a preferred aspect of the process of the present invention, in step a), after the addition of the base, the reaction is carried out at a temperature comprised in the range from -30° C to 20° C.

According to a more preferred aspect of the process of the present invention, in step a), after the addition of the base, the reaction is carried out at a temperature comprised in the range from -20° C to 0° C.

According to another preferred aspect of the process of the present invention, in step a), after the addition of the base, the reaction is carried out over a period comprised between 30 minutes and 6 hours.

According to a more preferred aspect of the process of the present invention, in step a), after the addition of the base, the reaction is carried out over a period comprised between 1 hour and 4 hours.

According to an even more preferred aspect of the process of the present invention, in step a) the addition of the base is carried out over a period comprised between 30 minutes and 5 hours at a temperature comprised in the range from -30° C to 20° C and, after the addition of the base, the reaction is carried out over a period comprised between 30 minutes and 6 hours at a temperature comprised in the range from -30° C to 20° C.

According to an even more preferred aspect of the process of the present invention, in step a) the addition of the base is carried out over a period comprised between 30 minutes and 1 hour at a temperature comprised in the range from -20° C to 0° C and, after the addition of the base, the reaction is carried out over a period comprised between 1 hour and 4 hours at a temperature comprised in the range from 10° C to 20° C.

According to a preferred aspect of the process of the present invention, in step a) the base added is comprised in the range from 2 molar equivalents to 10 molar equivalents with respect to compound of formula (3).

According to a more preferred aspect of the process of the present invention, in step a) the base added is comprised in the range from 2 molar equivalents to 5 molar equivalents with respect to compound of formula (3).

Molar equivalent or equivalent (eq) means that the molar amount of a substance reacts with a molar amount of another substance in a given chemical reaction.

As intended herein, a salt or ionic compound is a chemical compound consisting of an assembly of positively charged ions (cations) and negatively charged ions (anions), which results in a compound with no net electric charge (electrically neutral). Thus, a monovalent, bivalent or trivalent salt is an ionic compound comprising one, two or three couples of charges (cation/anion).

According to a preferred aspect of the process of the present invention, in step a) the compound of formula (3) is a monovalent, bivalent, or trivalent salt.

According to a preferred aspect of the process of the present invention, in step a) the compound of formula (3) is a salt comprising anions that are part of strong acids.

Strong acids are acids that are completely or nearly completely ionized in their solutions. Hydrochloric acid, Hydrobromic acid, Hydroiodic acid, Sulfonic acid are examples of strong acids.

Therefore, according to a preferred aspect of the process of the present invention, compound of formula (3) is a salt of a strong acid selected among hydrochloric acid, hydrobromic acid, hydroiodic acid, sulfonic acid.

According to a more preferred aspect of the process of the present invention, in step a) the compound of formula (3) is a monovalent, bivalent, or trivalent salt comprising anions that are part of strong acids.

According to an even more preferred aspect of the process of the present invention, compound of formula (3) is a monohydrochloride salt, a dihydrochloride salt or a trihydrochloride salt.

According to a particularly preferred aspect of the process of the present invention, compound of formula (3) is a dihydrochloride salt.

According to the process of the present invention, in step a) by adding a base to a mixture comprising amino piperazine compound of formula (3): 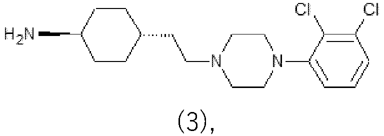 wherein said compound of formula (3) is in a salt form, and phosgene or a carbonic acid derivative of formula (10):

R-O-CO-Z (10),

wherein R is a C₁₋₆ straight or branched alkyl chain or a C₁₋₂ fully halogenated alky and Z is O-R or X, wherein R is as described above and X is a halogen, the isocyanate compound of formula (2): 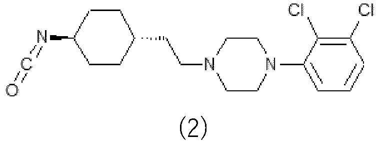 and/or compound of formula (11): 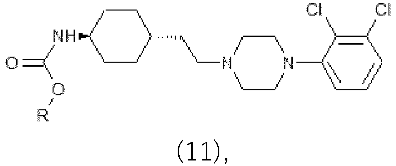 wherein R is as described above, is obtained. Compound of formula (2) and/or compound of formula (11) is then reacted with dimethylamine in step b) to obtain Cariprazine compound of formula (1).

According to a preferred aspect of the process of the present invention, the isocyanate compound of formula (2) and/or the compound of formula (11) is either isolated before carrying out step b) or reacted *in situ* in step b) without isolation.

According to a more preferred aspect of the process of the present invention, compound of formula (2) and/or compound of formula (11) are not isolated.

Therefore, according to a more preferred aspect of the process of the present invention, step a) and step b) are carried out as a one pot reaction.

In step b), compound of formula (2) and/or compound of formula (11) are reacted with dimethylamine. The reaction can be carried out by dosing dimethylamine or a dimethylamine solution over a mixture comprising compound of formula (2) and/or compound of formula (11).

In another aspect, the reaction can be carried out by dosing a mixture comprising compound of formula (2) and/or compound of formula (11) over a solution comprising dimethylamine.

According to a preferred aspect of the process of the present invention, therefore, in step b) the reaction of compound of formula (2) and/or compound of formula (11) with dimethylamine is performed either by dosing dimethylamine or a dimethylamine solution over the mixture comprising compound of formula (2) and/or compound of formula (11), or by dosing the mixture comprising compound of formula (2) and/or compound of formula (11) over dimethylamine or a dimethylamine solution.

Prior art process disclosed in WO2010070370 Example 3 employs dimethylamine as a solution in isopropanol.

It is well known in industrial manufacturing that any effort should be spent towards more sustainable processes. In this perspective, organic solvents should be replaced, as far as possible, with water.

It was surprisingly found that reaction of step b) according to the process of the present invention can be carried out using dimethylamine as aqueous solution instead of dimethylamine hydrochloride or dimethylamine as solution in isopropanol without negatively affecting process performances. As shown in Example 10, in fact, in all the cases where dimethylamine (DMA) was employed as aqueous solution following the present application process, a higher yield corrected for the product assay (HPLC wt%) was obtained (94% average corrected molar yield compared to 91% corrected molar yield obtained following WO2010070370 Example 3 process).

The process object of the present application, therefore, allows to replace the organic solvent employed for the dilution of dimethylamine with water, while, at the same time, assuring process performances are not affected and still higher than the performances obtained when applying prior art processes.

According to a preferred aspect of the process of the present invention, in step b) dimethylamine is employed as a solution in water.

According to a preferred aspect of the process of the present invention, therefore, in step b) dimethylamine is dimethylamine in aqueous solution.

The present invention is also addressed to a pharmaceutical composition comprising compound of formula (1) obtained by the process of the present application and at least one pharmaceutically acceptable excipient.

The present invention is therefore also addressed to a process for preparing a pharmaceutical composition comprising compound of formula (1), comprising the following steps:
a1) preparing compound of formula (1) according to the process of the present application;
b1) mixing compound of formula (1) obtained in step a1) with at least one pharmaceutically acceptable excipient.

The present invention is also addressed to a process for the preparation of compound of formula (2): 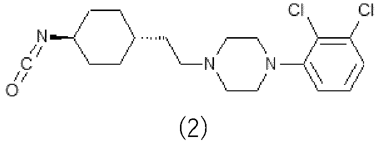 and/or compound of formula (11): 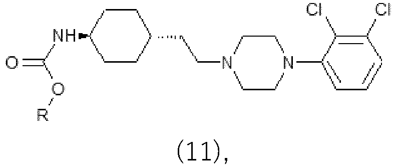 wherein R is a C₁₋₆ straight or branched alkyl chain or a C₁₋₂ fully halogenated alky and Z is O-R or X, wherein R is as described above and X is a halogen, comprising adding a base to a mixture comprising:
- amino piperazine compound of formula (3): 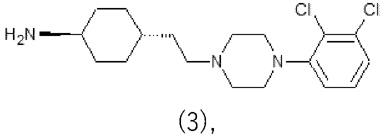 wherein said compound of formula (3) is in a salt form, and
- phosgene or a carbonic acid derivative of formula (10):

   R-O-CO-Z (10),

   wherein R is a C₁₋₆ straight or branched alkyl chain or a C₁₋₂ fully halogenated alky and Z is O-R or X, wherein R is as described above, and X is a halogen.

All the features and preferred aspects of the process of the present invention given above can be combined in each possible combination to carry out the claimed process.

### Experimental Section

Compound of formula (5) is commercially available, for example by abcr GmbH. Compound of formula (4) can be prepared, for example, according to Example 2 of WO2005012266 (page 19). Compound of formula (3) can be prepared, for example, according to Example 3 of WO2005012266 (page 19).

As intended herein, the term C₁-C₆ linear or branched alkyl, means one alkyl group characterized by one to six carbon atoms, such as methyl, ethyl, 1-propyl, 2-propyl, 1-butil, 2-butyl, 2-methyl-1-propyl, 2-methyl-2-propyl, 1-pentyl, 1-hexyl etcetera.

Room temperature (RT) means a temperature that is comprised in a range from 20 to 25° C, it is defined as comfortable temperature range indoors.

As intended herein, drying the wet material under *vacuum* means to heat the material to a given temperature for a given time while applying a reduced pressure. This operation is performed to remove residual solvents from the product.

As intended herein, to concentrate a mixture under *vacuum* means to heat the material to a given temperature for a given time while applying a reduced pressure. This operation is performed to remove solvents from the mixture.

Molar concentration or molarity is a measure of the concentration of a chemical species, in particular, of a solute in a solution, in terms of moles of substance per unit volume of solution. In chemistry, the most commonly used unit for molarity is the number of moles per Liter, having the unit symbol mol/L. A solution with a concentration of 1 mol/L is said to be 1 molar, commonly designated as 1 M.

The following abbreviation were used in the present document: DMA = dimethylamine; TEA = triethylamine; DIPEA = N,N-diisopropyl ethylamine; DCM = dichloromethane; THF = tetrahydrofuran; Me-THF = 2-methyl-tetrahydrofuran.

### Example 1: Preparation of compound of formula (1).

In a round bottom flask equipped with a half-moon stirrer, compound of formula (3) as dihydrochloride salt (5.0 g), triphosgene (1.8 g) and Me-THF (70 mL) are charged. The mixture is stirred at RT for 10 minutes and then cooled down to T = -20° C over 1.5 hours. TEA (5.9 g) is dosed over the mixture in 45 minutes, keeping the batch temperature at T = -20° C. After warming to RT for 1 hour to allow the reaction to reach completion, the mixture is cooled again to T = 0° C in 45 minutes and, keeping this temperature, an aqueous solution of DMA (2.6 g of a 40% wt solution) is dosed over 40 minutes into the mixture. After 4 hours stirring at T = 0° C, the batch is divided into two equal parts. Half of the organic solution is stored for analytical purposes. The second half of the solution (34.5 g) is diluted with water (10 mL) and the biphasic mixture is stirred at RT for 40 minutes and then heated to T=60° C for 1.5 hours. The resulting suspension is filtered at the same temperature, washing the wet cake with a pre-heated mixture of water (10 mL) and methanol (10 mL) at T = 55° C. The white solid obtained is dried under *vacuum* at T = 50° C, yielding 2.5 g of compound of formula (1). Molar Yield (not corrected for assay): 100%; HPLC assay: 92.6%wt; Molar Yield (corrected for assay): 93%.

### Example 2: Preparation of compound of formula (1).

In a reactor equipped with a propeller stirrer, compound of formula (3) as dihydrochloride salt (5.0 g), triphosgene (1.8 g) and Me-THF (70 mL) are charged. The mixture is stirred at RT for 5 minutes and then cooled down to T = -20° C over 40 minutes. TEA (5.9 g) is dosed over the mixture in 40 minutes, keeping the batch temperature at T = -20° C. After warming to RT for 1 hour to allow the reaction to reach completion, the mixture is cooled again to T = -20° C in 45 minutes and, keeping this temperature, an aqueous solution of DMA (30 mL of a 40% wt solution) is dosed over 35 minutes into the mixture. After 15 minutes stirring at T = -20° C, the batch is warmed to RT and stirred at this temperature for 1h and finally heated to T = 70° C. The resulting suspension is concentrated under reduced pressure to 40 mL residual volume, stirred at T = 60° C for additional 10 minutes and then filtered. The wet cake is washed with a pre-heated mixture of water (10 mL) and methanol (10 mL) at T = 55° C. The white solid obtained is dried under *vacuum* at T = 50° C, yielding 4.9 g of compound of formula (1). Molar Yield (not corrected for assay): 98%; HPLC assay: 95.6%wt; Molar Yield (corrected for assay): 94%.

### Example 3: Preparation of compound of formula (1).

In a reactor equipped with an impeller stirrer, compound of formula (3) as dihydrochloride salt (5.0 g), triphosgene (1.8 g) and Me-THF (70 mL) are charged. The mixture is stirred at RT for 25 minutes and then cooled down to T = -20° C over 35 minutes. TEA (5.9 g) is dosed over the mixture in 35 minutes, keeping the batch temperature at T = -20° C. After warming to RT for 4 hours to allow the reaction to reach completion, the mixture is cooled again to T = -20° C in 40 minutes and, keeping this temperature, an aqueous solution of DMA (30 mL of a 40% wt solution) is dosed over 1.5 hours into the mixture. After 30 minutes stirring at T=-20° C, the batch is warmed to RT and then heated to T = 45° C. The resulting suspension is concentrated under reduced pressure to 30 mL residual volume, and then diluted with a mixture of water (15 mL) and isopropanol (5 mL). After concentration to 25 mL residual volume under reduced pressure, the batch is diluted again with water (15 mL) and concentrated to 30 mL residual volume under the same conditions. After adding isopropanol (10 mL) the mixture is heated to T = 60° C for 15 minutes and then filtered. The wet cake is washed with a pre-heated mixture of water (20 mL) and isopropanol (20 mL) at T = 60° C. The white solid obtained is dried under *vacuum* at T = 50° C, yielding 4.9 g of compound of formula (1). Molar Yield (not corrected for assay): 98%; HPLC assay: 97.8%wt; Molar Yield (corrected for assay): 96%.

### Example 4: Preparation of compound of formula (1).

In a reactor equipped with an impeller stirrer, compound of formula (3) as dihydrochloride salt (5.0 g), triphosgene (2.75 g) and Me-THF (70 mL) are charged. The mixture is stirred at RT for 10 minutes and then cooled down to T = -20° C over 50 minutes. TEA (5.9 g) is dosed over the mixture in 40 minutes, keeping the batch temperature at T = -20° C. After warming to RT for 1 hour to allow the reaction to reach completion, the mixture is cooled again to T = -20° C in 50 minutes and, keeping this temperature, an aqueous solution of DMA (30 mL of a 40% wt solution) is dosed over 40 minutes into the mixture. The batch is divided into two equal portions. One of the two portions is stored for analytical purposes. The remaining portion is cooled to T = 0° C and then filtered. The wet cake is washed with a mixture of water (20 mL) and methanol (20 mL) at T = 0° C. The white solid obtained is dried under *vacuum* at T = 50° C, yielding 2.4 g of compound of formula (1). Molar Yield (not corrected for assay): 100%; HPLC assay: 97.4%wt; Molar Yield (corrected for assay): 97%.

### Example 5: Preparation of compound of formula (1).

In a reactor equipped with an impeller stirrer, compound of formula (3) as dihydrochloride salt (10.0 g), triphosgene (3.6 g) and Me-THF (140 mL) are charged. The mixture is stirred at RT for 15 minutes and then cooled down to T=-20° C over 45 minutes. TEA (11.8 g) is dosed over the mixture in 55 minutes, keeping the batch temperature at T=-20° C. After warming to RT for 1 hour to allow the reaction to reach completion, the mixture is cooled again to T = -20° C in 30 minutes and, keeping this temperature, an aqueous solution of DMA (60 mL of a 40% wt solution) is dosed over 50 minutes into the mixture. After 25 minutes stirring at T = -20° C the batch is concentrated under reduced pressure to 60 mL residual volume. The mixture is stripped under the same conditions two times with water (2 x 30 mL), then diluted with isopropanol (30 mL) and heated to T = 60° C. After 25 minutes stirring at this temperature, the suspension is filtered. The wet cake is washed with a mixture of water (40 mL) and isopropanol (40 mL) pre-heated to T = 60° C. The white solid obtained is dried under *vacuum* at T = 50° C, yielding 9.25 g of compound of formula (1). Molar Yield (not corrected for assay): 93%; HPLC assay: 99.2%wt; Molar Yield (corrected for assay): 92%.

### Example 6: Preparation of compound of formula (1).

In a reactor A equipped with an impeller stirrer, compound of formula (3) as dihydrochloride salt (5.0 g), triphosgene (1.8 g) and Me-THF (70 mL) are charged. The mixture is stirred at RT for 15 minutes and then cooled down to T = -20° C over 35 minutes. TEA (5.9 g) is dosed over the mixture in 45 minutes, keeping the batch temperature at T = -20° C. The mixture is then warmed to RT for 4 hours, to allow the reaction to reach completion. In a separate reactor B, an aqueous solution of DMA (30 mL of a 40% wt solution) is charged and adjusted to T = -10° C. The mixture of reactor A is transferred under nitrogen pressure into reactor B, maintaining this temperature, followed by a Me-THF wash of reactor A (10 mL). Thereafter, the batch is concentrated under reduced pressure to 30 mL residual volume. The mixture is stripped under the same conditions two times with water (2 x 15 mL), then diluted with isopropanol (15 mL) and heated to T = 60° C. After 25 minutes stirring at this temperature, the suspension is filtered. The wet cake is washed with a mixture of water (20 mL) and isopropanol (20 mL) pre-heated to T = 60° C. The white solid obtained is dried under *vacuum* at T = 50° C, yielding 4.7 g of compound of formula (1). Molar Yield (not corrected for assay): 94%; HPLC assay: 100.4%wt; Molar Yield (corrected for assay): 94%.

### Example 7: Preparation of compound of formula (1), Reworked of Method C of patent application WO2005012266.

In a round bottom flask equipped with a half-moon stirrer, compound of formula (3) as dihydrochloride salt (5.0 g) and DCM (500 mL) are charged, followed by TEA (5.0 g). After 15 min at RT, a solution of triphosgene (1.28 g) in DCM (5.0 mL) is dosed over 1 hour. After 1 hour stirring at RT, dimethylamine hydrochloride (4.74 g) is added to the mixture, followed by TEA (6.0 g). After 20 hours stirring at RT, the mixture is filtered, washing the wet cake with water (20 mL). The solid is dried under *vacuum* at T=45° C, yielding 4.85 g of compound of formula (1). Molar Yield (not corrected for assay): 79%; HPLC assay: 58.3%wt; Molar Yield (corrected for assay): 46%.

### Example 8: Preparation of compound of formula (1), Reworked of Example 3 of patent application WO2010070370.

In a reactor A equipped with a propeller stirrer, compound of formula (3) as dihydrochloride salt (5.0 g) is charged, followed by a solution of TEA (7.0 g) in DCM (97 mL). After 1 hour stirring at RT, the thick suspension is cooled down to T=5° C. In the meanwhile, in a different reactor B equipped with an impeller stirrer, a solution of triphosgene (3.8 g) in DCM (39 mL) is prepared and cooled to T=-10° C. The thick suspension of reactor A is transferred to reactor B over 15 minutes and the mixture is allowed to react for 1 hour at T=-10° C. In a different reactor C equipped with an impeller stirrer, a solution of dimethylamine in isopropanol (46 mL of a 2M solution) is charged and cooled down to T=-10° C. The suspension of reactor B is transferred into reactor C and the resulting mixture is warmed to T=-5° C. After 1 hour stirring at T=-5° C, the batch is diluted with water (78 mL) and, keeping the same temperature, an aqueous solution of hydrochloric acid (3.2 mL of a 32%wt solution) are added. The mixture is then warmed to RT and concentrated under reduced pressure to 100 mL residual volume. The residue is diluted with water (54 mL) and concentrated again under reduced pressure to 130 mL residual volume. After 20 minutes stirring at RT, the resulting suspension is filtered, and the wet cake is dried under *vacuum* at T=50° C, yielding 4.9 g of compound of formula (1). Molar Yield (not corrected for assay): 98%; HPLC assay: 93.3%wt; Molar Yield (corrected for assay): 91%.

### Example 9: Comparison between prior art and present application process performances.

In the table below are collected the molar yield, HPLC assay and molar yield corrected for assay for the conversion of compound of formula (3) to Cariprazine compound of formula (1) following prior art processes reported in WO2005012266 and in WO2010070370 and following the present application process. The entry highlighted in the table (Example# Avg) represents the average values obtained when applying the process of the present application.

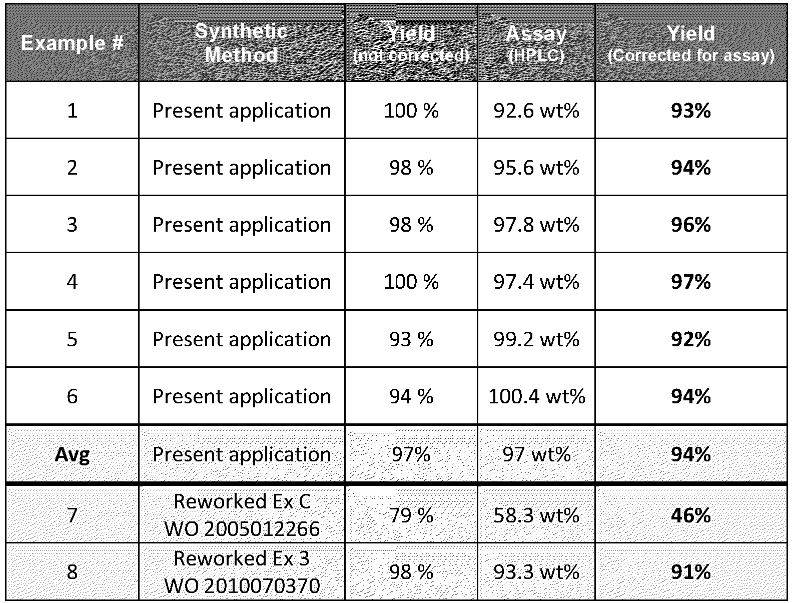

The results reported in the table clearly show that the present application process allows to obtain Cariprazine with a considerably higher yield corrected for the assay with respect to the processes known in the prior art. In addition, the process of the present invention is suitable for industrial manufacturing, as it does not comprise the transfer of thick suspensions which is not feasible in standard industrial equipment.

### Example 10: Comparison between prior art and present application process performances.

In the table below are collected the amount of triphosgene charged, the solvent used for the reaction and the source of DMA employed, together with the molar yield corrected for assay for the conversion of compound of formula (3) to Cariprazine compound of formula (1) following prior art processes reported in WO2005012266 and in WO2010070370, and following the present application process.

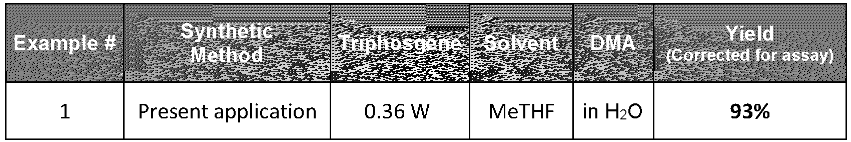

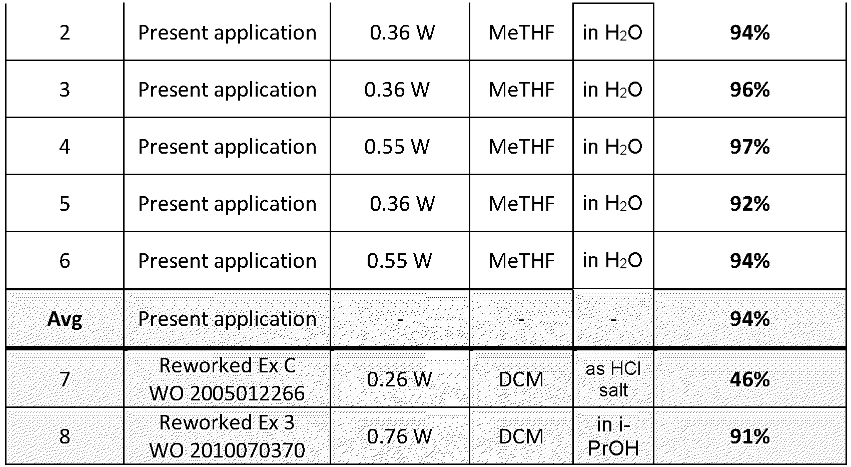

The results reported in the table clearly show that the present application process allows to reduce the amount of the toxic reagent triphosgene, since the process of WO2005012266, where only 0.26 W of triphosgene are used, is characterized by an extremely low yield (46%). By reducing the amount of triphosgene from 0.76 W, which is the amount of reactant employed in WO2010070370, to 0.55 W or 0.36 W, which is the amount employed according to the present invention process, conversely, the process performance in terms of yield corrected for assay are even improved (94% average yield is obtained following the present application process, while 91% yield is obtained following the process disclosed in WO2010070370).

Moreover, the present application process allows to replace the toxic and environmentally polluting DCM with Me-THF as reaction solvent.

Specifically, Me-THF can be produced from biomass feedstock, such as levulinic acid or hydrogenation of furfural, which are produced from lignocellulosic biomass. Being derived from renewable resources, Me-THF is a promising alternative solvent in the search for environmentally benign synthesis strategies.

In addition, the problem related to the transfer of the thick suspension of WO2010070370 is solved.

The use of DMA as an aqueous solution instead of a solution in isopropanol is an additional contribution to the greenness of the present application process.

It clearly appears, therefore, that the process of the present invention is also more environmentally sustainable with respect to prior art methods.

## Claims

1. Process for the preparation of Cariprazine compound of formula (1): 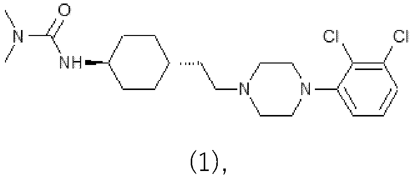 comprising the following steps:
a) adding a base to a mixture comprising:
- amino piperazine compound of formula (3): 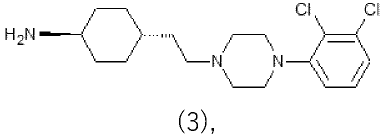 wherein said compound of formula (3) is in a salt form, and
- phosgene or a carbonic acid derivative of formula (10):
R-O-CO-Z (10),
wherein R is a C₁₋₆ straight or branched alkyl chain or a C₁₋₂ fully halogenated alky and Z is O-R or X, wherein R is as described above and X is a halogen,
to give an isocyanate compound of formula (2): 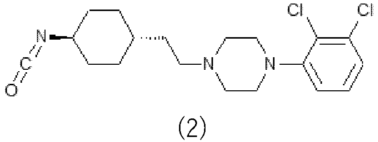 and/or compound of formula (11): 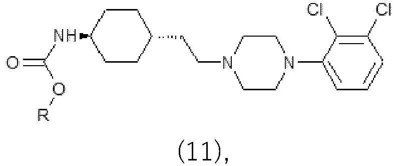 wherein R is as described above;
b) reacting compound of formula (2) and/or compound of formula (11) obtained in step a) with dimethylamine to give Cariprazine compound of formula (1).

2. Process according to claim 1, wherein in step a) the base is triethylamine or diisopropylethylamine.

3. Process according to any one of the claims 1 or 2, wherein in step a) the base is added over a period comprised between 30 minutes and 5 hours.

4. Process according to any one of the claims from 1 to 3, wherein in step a) the base is added at a temperature comprised within the range from -30° C to 20° C.

5. Process according to any one of the claims from 1 to 4, wherein in step a) the carbonic acid derivative (10) is triphosgene or diphosgene.

6. Process according to any one of the claims from 1 to 5, wherein step a) is carried out in a solvent selected from the list comprising 2-methyl-tetrahydrofuran, tetrahydrofuran, acetone, acetonitrile.

7. Process according to anyone of the claims from 1 to 6, wherein compound of formula (3) is a salt of a strong acid selected among hydrochloric acid, hydrobromic acid, hydroiodic acid, sulfonic acid.

8. Process according to anyone of the claims from 1 to 7, wherein compound of formula (3) is a monovalent, bivalent, or trivalent salt.

9. Process according to anyone of the claims from 1 to 8, wherein compound of formula (3) is in salt form as monohydrochloride salt, a dihydrochloride salt or a trihydrochloride salt.

10. Process according to anyone of the claims from 1 to 9, wherein compound of formula (3) is a dihydrochloride salt.

11. Process according to any one of the claims from 1 to 10, wherein the isocyanate compound of formula (2) and/or the compound of formula (11) is either isolated before carrying out step b) or reacted *in situ* in step b) without isolation.

12. Process according to any one of the claims from 1 to 11, wherein in step b) the reaction of compound of formula (2) and/or compound of formula (11) with dimethylamine is performed either by dosing dimethylamine or a dimethylamine solution over the mixture comprising compound of formula (2) and/or compound of formula (11), or by dosing the mixture comprising compound of formula (2) and/or compound of formula (11) over dimethylamine or a dimethylamine solution.

13. Process according to any one of the claims from 1 to 12, wherein in step b) dimethylamine is dimethylamine in aqueous solution.

14. Process for preparing a pharmaceutical composition comprising compound of formula (1), comprising the following steps:
a1) preparing compound of formula (1) according to the process of any one of the claims from 1 to 13;
b1) mixing compound of formula (1) obtained in step a1) with at least one pharmaceutically acceptable excipient.

15. Process for the preparation of compound of formula (2): 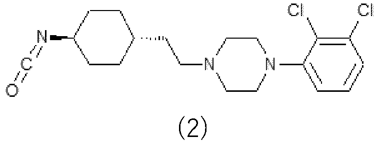 and/or compound of formula (11): 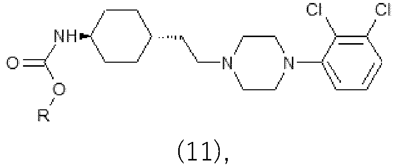 wherein R is a C₁₋₆ straight or branched chain alkyl or a C₁₋₂ fully halogenated alky and Z is O-R or X, wherein R is as described above and X is a halogen, comprising adding a base to a mixture comprising:
- amino piperazine compound of formula (3): 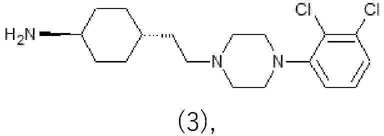 wherein said compound of formula (3) is in a salt form, and
- phosgene or a carbonic acid derivative of formula (10):
R-O-CO-Z (10),
wherein R is a C₁₋₆ straight or branched alkyl chain or a C₁₋₂ fully halogenated alky and Z is O-R or X, wherein R is as described above, and X is a halogen.
